(19)

Europäisches
Patentamt
European
Patent Office
Office européen
des brevets

(11) **EP 2 725 967 B1**

(12) **EUROPEAN PATENT SPECIFICATION**

(45) Date of publication and mention
of the grant of the patent:
**05.08.2020 Bulletin 2020/32**

(51) Int Cl.:
***A61B 5/00*** *(2006.01)*

(21) Application number: **12740211.3**

(86) International application number:
**PCT/IB2012/053133**

(22) Date of filing: **21.06.2012**

(87) International publication number:
**WO 2013/001423 (03.01.2013 Gazette 2013/01)**

(54) **AN APPARATUS FOR OPTICAL ANALYSIS OF AN ASSOCIATED TISSUE SAMPLE**

VORRICHTUNG ZUR OPTISCHEN ANALYSE EINER GEWEBEPROBE

APPAREIL POUR ANALYSE OPTIQUE D'UN ÉCHANTILLON DE TISSU ASSOCIÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.06.2011 EP 11171666**

(43) Date of publication of application:
**07.05.2014 Bulletin 2014/19**

(73) Proprietor: **Koninklijke Philips N.V.
5656 AG Eindhoven (NL)**

(72) Inventors:
• **MUELLER, Manfred
NL-5656 AE Eindhoven (NL)**
• **HENDRIKS, Bernardus Hendrikus Wilhelmus
NL-5656 AE Eindhoven (NL)**
• **BIERHOFF, Waltherus Cornelis Jozef
NL-5656 AE Eindhoven (NL)**
• **LUCASSEN, Gerhardus Wilhelmus
NL-5656 AE Eindhoven (NL)**
• **HORIKX, Jeroen Jan Lambertus
NL-5656 AE Eindhoven (NL)**
• **NACHABE, Rami
NL-5656 AE Eindhoven (NL)**
• **VAN DER VOORT, Marjolein
NL-5656 AE Eindhoven (NL)**

(74) Representative: **Philips Intellectual Property &
Standards
High Tech Campus 5
5656 AE Eindhoven (NL)**

(56) References cited:
**US-A1- 2008 097 174     US-B1- 6 219 566
US-B1- 6 571 118**

• **ANTHONY KIM ET AL: "Quantification of in vivo
fluorescence decoupled from the effects of tissue
optical properties using fiber-optic spectroscopy
measurements", JOURNAL OF BIOMEDICAL
OPTICS, vol. 15, no. 6, 1 January 2010
(2010-01-01), pages 067006-1-067006-12,
XP55029337, ISSN: 1083-3668, DOI:
10.1117/1.3523616**
• **NARASIMHAN RAJARAM ET AL: "Lookup
table-based inverse model for determining
optical properties of turbid media", JOURNAL OF
BIOMEDICAL OPTICS, vol. 13, no. 5, 1 January
2008 (2008-01-01), page 050501, XP55029285,
ISSN: 1083-3668, DOI: 10.1117/1.2981797 cited in
the application**

**Description**

FIELD OF THE INVENTION

The present invention relates to an apparatus for optical analysis of an associated tissue sample, and more specifically to an apparatus, a method and a computer program for optical analysis of an associated tissue sample.

BACKGROUND OF THE INVENTION

Fluorescence spectroscopy can give crucial information about the state of biological tissue, e.g. for cancer detection. However, the interplay of scattering and absorption usually results in severe distortion of the intrinsic fluorescence spectra.

The reference EP 1 942 335 describes an apparatus for optically measuring tissue, the apparatus comprises a radiation source that illuminates a region of interest in tissue to be measured with incident radiation, an optical system that collects scattered and fluorescent light from the tissue at a plurality of wavelengths, a detector system that senses the collected light and provides fluorescence data and scattered light data as a function of wavelength; and a data processor that determines the characteristic of the region of interest with the fluorescence data and the scattered light data.

US 6,219,566 B1 describes a method of measuring concentration of luminescent materials in turbid media. One optical fiber emits light, while two optical fibers at different distances from the light emitting optical fiber, serve to detect light reflected from the medium under test. A ratio between detected fluorescence and reflectance is finally calculated.

"Quantification of in vivo fluorescence decoupled from the effects of tissue optical properties using fiber-optic spectroscopy measurements", Journal of Biomedical Optics, Vol. 15, No. 6, 1 January 2010, pages 067006-1-067006-12, ISSN: 1083.3668, DOI: 10.1117/1.3523616, describes a method for in situ quantification of tissue fluorescence. Based on fluorescence and reflectance measurements, a method for correcting the fluorescence data based on the reflectance measurements is described.

US 6,571,118 B1 describes probe for combined fluorescence and reflectance spectroscopy. A ring of fluorescence collecting fibers are arranged outside a circle of fluorescence excitation fibers. A set of reflectance excitation and reflectance collecting fibers are arranged outside the ring of fluorescence collecting fibers.

US 2008/097174 A1 describes a method of determining a measure of a tissue state based on fluorescence measurement. It is described that the measured fluorescence can be corrected to arrive at an intrinsic fluorescence by knowledge of measured reflectance.

There are thus methods to take the above described distortions into account. However, there remains a desire in the field to obtain further improvements in the optical measurements and/or the fluorescence spectra achieved.

Hence, an improved apparatus which could aid in improving the optical measurements and/or the fluorescence spectra achieved would thus be advantageous, and in particular a more efficient and/or reliable apparatus would be advantageous.

SUMMARY OF THE INVENTION

It is a further object of the present invention to provide an alternative to the prior art.

In particular, it may be seen as an object of the present invention to provide an apparatus for optical analysis of an associated tissue sample that solves the above mentioned problems of the prior art by improving the optical measurements and/or the fluorescence spectra achieved.

Thus, the above described object and several other objects are intended to be obtained in a first aspect of the invention by providing an apparatus for optical analysis of an associated tissue sample as defined in claim 1.

The invention is particularly, but not exclusively, advantageous for determining a third set of data representative of an intrinsic fluorescence spectrum of the associated tissue sample based on the first and second set of data. It may be seen as advantageous, that the first and second sets of data are determined using light emitters and light collectors which are spaced apart with different distances, so as to enable that each one of the measurements - of the first and second set of data - may be optimized. It may thus be seen as the gist of the invention that the first and second sets of data are determined using light emitters and light collectors which are spaced apart with different distances and/or under different geometries, so as to enable that each one of the measurements - of the first and second set of data - may be optimized while still retaining the ability to use the first set of data to correct distortions in the second set of data.

It is understood that the first distance d1 between the first light emitter and the second light collector is substantially larger than a second distance d2 between the second light emitter and the second light collector, such as 5 % larger, such as 10 % larger, such as 20 % larger, such as 50 % larger, such as 75 % larger, such as 90 % larger than a second distance d2 between the second light emitter and the second light collector.

'Light' is to be broadly construed as electromagnetic radiation comprising wavelength intervals including visible, ultraviolet (UV), near infrared (NIR), infra red (IR), X-ray. The term optical is to be understood as relating to light.

A 'fluorescence spectrum' or a 'Reflectance Spectrum', are each understood to be an optical spectrum, which is understood to be information related to a plurality of wavelengths of light, such as an intensity parameter, an absorption parameter, a scattering parameter or a transmission parameter given for a plurality of wavelengths of light. A continuous spectrum represents spectral information, but it is further understood, that information related to light at discrete wavelengths may represent an optical spectrum.

A 'spectrometer' is understood as is common in the art and is suited for measuring light within a plurality of different wavelength ranges, so as to enable the measurement of an optical spectrum. It is understood that the spectrometer comprises at least one optical detector so as to be able to detect incident light. It is furthermore understood, that the spectrometer comprises means for selecting wavelengths, such as transmission filters or gratings. Alternatively, wavelength specific light sources, such as light emitting diodes or LASERs, may be used or wavelength specific optical detectors may be used. A spectral filtration may occur at different places in the system, for instance it may occur between the second light source and an interventional device, it may occur in the interventional device, or it may occur between the interventional device and the optical detector. It is also understood that 'spectrometer' may refer to set of spectrometers, light guides and possibly beam splitters designed in such a way that different spectrometers measure different parts of the optical spectrum. It is noted that throughout this application, 'spectrometer' is used interchangeably with 'optical spectrometer' and 'detector' is used interchangeably with 'optical detector'.

In the present context 'intrinsic fluorescence' is defined as the fluorescence that is due only to fluorophores, without interference from the absorbers and scatterers that may be present in the associated tissue sample.

As will be apparent from the various embodiments and the detailed description, the first and second light emitter and the first and second light collector may represent two, three or four different optical elements, since an optical element embodying anyone of the first and second light emitter and the first and second light collector may double or triple function so as to also embody another one or two of the first and second light emitter and the first and second light collector. For example in a specific embodiment a distal end of a light guide may function as both first light emitter, second light emitter and second light collector, and another light guide may function as first light collector. In that specific embodiment only two light guides are required to represent the first and second light emitter and the first and second light collector.

The invention can be used in the field of oncology, or other healthcare applications where the determination of tissue type is relevant. It might also be applied to any medical field where optical biopsies or optical tissue discrimination might be used.

The apparatus may be applicable for real-time intra-operative needle localization and/or ablation monitoring to improve ablation efficacy and disease free survival. It is noted, that concentrations of various chromophores, which may be determined based on the intrinsic fluorescence spectrum, may be indicative for discriminating certain types of tissue, such as discriminating pathological tissue from normal tissue. As other examples of applications where embodiments of the present invention may be used include applications in any field where accurate determination of concentrations or presence of chromophores is important. It may thus be used in fields where the determination of a quality parameter of, for example, food is relevant, such as determining the type of tissue in a meat product. It might also be used in cases where it is important to determine the type of tissue at the tip of a needle or probe, e.g. for taking biopsies of a certain type of tissue or for injecting a substance into a certain type of tissue.

According to an embodiment of the invention, the apparatus is further comprising:

- a database comprising a predetermined table of correction factors which enables determination of a third set of data being based on the first set of measured data and the second set of measured data,

and the processor further being arranged for:

- accessing the database, and

wherein the determining the third set of data representative of an intrinsic fluorescence spectrum of the associated tissue is furthermore based on the predetermined table of correction factors.

An advantage of this embodiment might be, that although it might appear, that the subsequent analysis of the data, which result in the third set of data, might be more complicated, the inventors of the present invention have made the basic insight that having established a predetermined table of correction factors enables an improved, fast, precise and reliable analysis even though the first and second set of data are determined using light emitters and light collectors which are spaced apart with different distances d1 and d2. Thus, the predetermined table of correction factors may enable an improved, fast, precise and reliable analysis.

The predetermined table of correction factors may be provided beforehand by calculation, such as by using the Monte Carlo technique, or measured, such as by using phantoms.

According to an embodiment of the invention, there is provided an apparatus wherein each one of the first light emitter, the first light collector, the second light emitter and the second light collector may be a distal end of a light guide.

A light guide is to be understood as is common in the art, and denotes an optical element through which light may guided. An advantage of using light guides may be that the generation or detection of light need not take place at the first light emitter, the first light collector, the second light emitter and the second light collector, so that the light source and the spectrometer comprising the detector may be placed away from the distal ends of the light guides.

According to another embodiment of the invention, there is provided an apparatus wherein the first light emitter, the first light collector, the second light emitter and the second light collector are comprised within an interventional device.

An 'interventional device' is understood to be an elongated body which is suited for entering tissue and/or body cavities. Interventional device may include any one of an endoscope, a catheter, a needle, a cannula, a biopsy needle and a drain. By integrating the first light emitter, the first light collector, the second light emitter and the second light collector in an interventional device, inspection of the optical characteristics of the associated tissue sample is facilitated in manner where only the interventional device needs to be placed adjacent to the associated tissue sample.

According to another embodiment of the invention, there is provided an apparatus wherein the first distance between the first light emitter and the first light collector is more than 1 mm, such as more than 1.25 mm, such as more than 1.5 mm, such as more than 1.75 mm, such as more than 2 mm, such as more than 2.25 mm, such as more than 2.5 mm, such as more than 3 mm, such as more than 5 mm. It is understood that the first distance between the first light emitter and the first light collector may be defined as a centre-to-centre distance, i.e., the distance from the centre of the first light emitter, such as the centre of the distal end of the first emitting light guide, to the centre of the first light collector, such as the centre of the distal end of the first collecting light guide.

According to another embodiment of the invention, there is provided an apparatus wherein the second distance between the second light emitter and the second light collector is less than 1 mm, such as less than 0.9 mm, such as less than 0.75 mm, such as less than 0.6 mm, such as less than 0.5 mm, such as less than 0.25 mm, such as less than 0.10 mm, such as substantially equal to 0 mm, such as equal to 0 mm. It is understood that the second distance between the second light emitter and the second light collector may be defined as a centre-to-centre distance, i.e., the distance from the centre of the second light emitter, such as the centre of the distal end of the second emitting light guide, to the centre of the second light collector, such as the centre of the distal end of the second collecting light guide. The case of having said distance equal 0 mm corresponds to the second light emitter and the second light collector being coincident, which may for example be the case if the second light emitter and the second light collector are one and the same distal end of a light guide, which is optically connected to both the light source and the spectrometer comprising the detector, and according to another embodiment of the invention, there is thus provided an apparatus wherein the second light emitter and the second light collector coincide, such as the second light emitter and the second light collector being one and the same distal end of a light guide. A possible advantage of this embodiment is that it maximizes the intensity of the fluorescent light and therefore the signal-to-noise ratio of the fluorescent spectrum. A possible further advantage might be that fewer elements are needed, for example, one light guide acting as both second light emitter, second light collector and first light emitter may together with another light guide acting as first light collector suffice in a possible embodiment. In that case, only two light guides are needed.

According to another embodiment of the invention, there is provided an apparatus wherein the first light emitter coincides with the second light emitter, such as the first light emitter being the same distal end of a light guide as the second light emitter.

According to another embodiment of the invention, there is provided an apparatus wherein a smallest distance d12 between the first light emitter and the first light collector, respectively, and the second light emitter and the second light collector is smaller than the first distance d1 between the first light emitter and the first light collector. A possible advantage of this embodiment may be, that substantially the same volume of the associated tissue sample is analysed, so that the even an inhomogeneous (with respect to a length scale of the order of dl) sample may be correctly analyzed.

According to another embodiment of the invention, there is provided an apparatus wherein the first light collector coincides with the second light collector, such as the first light collector being the same light collector as the second light collector, such as the first light collector being the same light guide as the second light collector. A possible advantage of this arrangement is that a single light collector may act as both the first light collector and the second light collector, and in this way the number of light collectors is reduced from two to one. Furthermore, using the same light collector as both first and second light collector may enable using the same optical spectrometer comprising the detector for obtaining both the first and second set of data, such as a fluorescence spectrum and a DRS spectrum, without the need for additional optics.

According to the first aspect, there is provided an apparatus wherein the processor is further arranged for determining a wavelength-dependent set of scattering and/or absorption coefficient from the first set of data.

According to an embodiment, the processor is further arranged for determining a set of scattering and/or absorption coefficients from the first set of data, wherein each of the scattering and/or absorbtion coefficients within the set of scattering and/or absorption coefficients correspond to a specific wavelength.

The scattering and absorption may be relevant on their own, as they represent information regarding the associated tissue sample. Furthermore, they are used to determine a distortion parameter. In the present context 'distortion parameter' is understood to depend on the contribution from scattering and absorption, and to be representative of scattering and absorption. This distortion parameter will be dependent on scattering and absorption at the fluorescence emission wavelelength but may in addition also depend on the scattering and absorption at the fluorescence excitation wavelength. It will be readily understood that the 'distortion parameter' is not limited to being a single number, but may be described as a number, a vector, a matrix, a table or a mathematical function, so as to enable the 'distortion parameter' to describe the distortion contributions from scattering and absorption for a number of constituents, such as biomolecules, across a number of wavelengths. It is noted that a possible advantage of knowing the distortion parameter may be that it renders it possible to take the distortion parameter into account, such as the distortion parameter determined from the first set of measured data enables removal of the effects of scattering and absorption from the second set of measured data. For example, an algorithm for disentangling contributions from scattering, absorption and fluorescence in a fluorescence spectrum of one or more different optically active constituents, such as chromophores, in a sample may not be able to correctly disentangle the contributions and correctly quantify the constituents if distortion (such as scattering and absorption) is present in the sample, unless the algorithm determines the distortion parameter and takes it into account. The distortion parameter may be a parameter enabling determination of intrinsic fluorescence in a fluorescence spectroscopy spectrum where the intrinsic fluorescence is entangled with the effects of scattering and/or absorption. In a particular embodiment the distortion parameter is based on any one of: scattering, absorption, a probe specific function, algorithm and/or constant and the anisotropy parameter of the associated tissue sample.

According to another embodiment of the invention, there is provided an apparatus wherein the database further comprises:

- predetermined fourth set of data representative of a predetermined fluorescence spectrum, and wherein the processor is further arranged for
- determining a first parameter being indicative of a concentration of a biomolecule in the associated tissue sample based on the third set of data and the fourth set of data.

According to another embodiment of the invention, there is provided an apparatus wherein the database comprises predetermined data representative of an optical spectrum. Having predetermined data representative of an optical spectrum stored in the database may be beneficial for determining from the measured data, such as from the first set of measured data and/or from the second set of measured data, a first parameter respectively a second parameter being indicative of a concentration of a biomolecule in the associated tissue sample. The predetermined data may be representative of spectra of a tissue type, or the predetermined data may be representative of an optical spectrum of a chromophore expected to be in the associated tissue sample, or predetermined data may be representative of the emission spectra of a fluorophore upon excitation with light of a given wavelength, which may be useful, e.g., as an input parameter in a mathematical model. The predetermined optical spectra may include spectra which have been calculated theoretically, such as by mathematical models, or spectra which have been measured on phantoms, such as samples prepared by mixing constituents expected to be in the associated tissue sample. Multivariate analysis is commonly known in the art and understood to include Principal Components Analysis (PCA) and least squares discriminant analysis.

According to another embodiment of the invention, there is provided an apparatus wherein the apparatus further comprises any one of: a light source for providing therapeutic light and/or an ultrasound unit. A possible advantage of providing a therapeutic light source is that it enables therapy using light. An advantage of providing an ultrasound unit may be that it enables ablation, such as radio frequency ablation or imaging.

The invention further relates to a method for optical analysis of an associated tissue sample as defined in claim 11.

This aspect of the invention is particularly, but not exclusively, advantageous in that the method according to the present invention may be implemented using an apparatus according to the first aspect of the invention.

The method may be carried out in the order listed, but the order in which the steps are listed or carried out is not important.

The method does not require interaction with a patient's body or involvement of a medical practitioner. In general, the invention is not about providing a diagnosis or treating a patient, but the invention may provide a technical solution for assisting a physician in reaching a diagnosis or treating a patient.

In a particular embodiment, the method may further include accessing a database with a predetermined table of correction factors, and determining the third set of data representative of an intrinsic fluorescence spectrum of the associated tissue sample based on the first and second set of data and the predetermined table of correction factors.

According to the second aspect, there is provided a method wherein a first volume of the associated tissue sample which is probed during the measuring of the first set of data substantially overlaps a second volume of the associated tissue sample which is probed during the measuring of the second set of data. A possible advantage of this invention may be, that substantially the same volume of the associated tissue sample is analysed, so that the even an inhomogeneous (with respect to a length scale of the order of dl) sample may be correctly analyzed.

According to an embodiment of the invention, there is provided a method wherein the second volume is substantially a subset of the first volume. In an alternative embodiment, at least 50 %, such as at least 80 %, of the second volume is a subset of the first volume.

According to another embodiment of the invention, there is provided a method further comprising determining a first parameter being indicative of a concentration of a biomolecule in the associated tissue sample, wherein the determination of the first parameter includes any one of:

- fitting the third set of data to a mathematical model,
- performing multivariate statistical analysis, such as PCA or partial least squares discriminant analysis, and
- accessing a look-up-table comprising a predetermined fourth set of data representative of a predetermined fluorescence spectrum.

According to this method for optical analysis of an associated tissue sample, the determination of the first parameter may include fitting the third data to a mathematical model. A mathematical model is in the present context understood to be a theoretical expression which for a given set of input parameters having influence on the optical spectrum, for example quantities of chromophores present, may as output yields data representative of an optical spectrum. Fitting is understood to be the process of adjusting the input parameters so as minimize a difference between a measured optical spectrum and a theoretically given optical spectrum. An advantage of this method, may be that it may be used to quantitatively estimate the first parameter.

According to a third aspect of the invention, the invention further relates to a computer program product.

The first, second and third aspect of the present invention may each be combined with any of the other aspects. These and other aspects of the invention will be apparent from and elucidated with reference to the embodiments described hereinafter.

BRIEF DESCRIPTION OF THE FIGURES

The apparatus, the method and the computer program for optical analysis of an associated tissue sample according to the invention will now be described in more detail with regard to the accompanying figures. The figures show one way of implementing the present invention and is not to be construed as being limiting to other possible embodiments falling within the scope of the attached claim set.

FIG 1        shows a schematic of an embodiment of the invention,
FIG 2        shows a perspective illustration of an embodiment of an interventional device,
FIGS 3-4    show a schematic depiction of the distal tip of an interventional device according to an embodiment of the invention,
FIG 5        shows absorption spectra for blood, water and lipid,
FIG 6        shows a schematic depiction of the top of a probe,
FIG 7        shows a measured DRS spectrum of a human breast,
FIG 8        is a graph of the scattering and absorption coefficients of the same human breast,
FIG 9        shows a measurement of the fluorescence spectrum of the same human human breast,
FIG 10      shows measured, intrinsic and true fluorescence spectra,
FIG 11      shows an example of a look-up table with values of $k$,
FIG 12      shows a top of a probe with various distances illustrated,
FIG 13      is a flow-chart illustrating a method according to an embodiment.

DETAILED DESCRIPTION OF AN EMBODIMENT

In the following and throughout this application "light guide" is used interchangeably with "fibre" or "optical fibre". Furthermore, "source light guide" is used interchangeably with "light emitter", and "detector light guide" is used interchangeably with "light collector", and "Reflectance Spectroscopy" is used interchangeably with "Diffuse Reflectance Spectroscopy" (DRS).

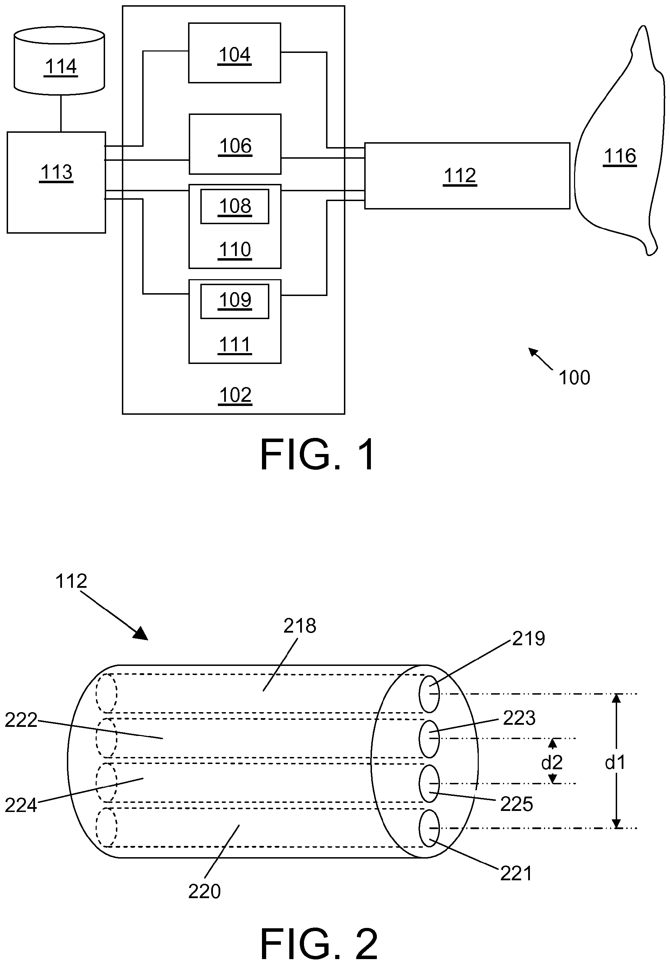

FIG 1 shows a schematic of an embodiment of the invention by showing an apparatus 100 according to an embodiment of the invention comprising an optical console 102 comprising a first light source 104, a second light source 106, a first spectrometer 110 comprising a first detector 108 and a second spectrometer 111 comprising a second detector 109. The figure furthermore shows an interventional device 112, where the interventional device 112 has four light guides, where the ends of the four light guides correspond to respectively a first light emitter, a first light collector, a second light emitter and a second light collector. It is understood that each light guide is an optical element, such as an optical waveguide, capable of guiding light from the light sources 104, 106 to a distal end of the interventional device so as to emit the light at the distal end of the interventional device and/or capable of guiding light back from the distal end of the interventional device to the spectrometers 110, 111 comprising the optical detectors 108, 109. The light guides enable light to enter an associated tissue sample 116 and the light guides further enable light leaving the associated tissue sample to be collected and led to the spectrometers 110, 111 comprising optical detectors 108, 109. The apparatus thus enables procurement of measured data representative of an optical spectrum of the associated tissue sample 116. The spectrometers 110, 111 comprising optical detectors 108, 109 may be controlled by processor 113 so as to acquire the measured data. The processor furthermore has access to a database 114, and the apparatus is further arranged to access the database 114, where the database 114 comprises a predetermined table of correction factors which enables determination of a third set of data being based on the first set of measured data and the second set of measured data. The processor 113 is arranged for receiving the first set of data, receiving the second set of data, accessing the database 114 with a predetermined table of correction factors, and determining a third set of data representative of an intrinsic fluorescence spectrum of the associated tissue sample based on the first and second set of data and the predetermined table of correction factors, wherein a first distance d1 (see FIG 2) between the distal ends of the first source and detector light guides is substantially larger than a second distance d2 (see FIG 2) between the distal ends of the second source and detector light guides.

In this embodiment the first light source 104 is a lamp suited for Reflectance Spectroscopy, such as Diffuse Reflectance Spectroscopy (DRS), and the second light source 106 is a LASER suited for fluorescence spectroscopy. In an alternative embodiment, there may be only a single light source, such as a single lamp which may then used in combination with a switchable filter serving to limit the range of frequencies emitted and thereby narrowing the bandwidth and thereby obtaining an appropriate bandwidth for doing fluorescence spectroscopy.

FIG 2 shows a perspective illustration of an embodiment of an interventional device 112, which interventional device comprises a first guide 218, a second guide 220, a third guide 222 and a fourth guide 224. The figure shows an exit position, which is thus a first light emitter 219, on a distal end of the first guide and an entry position, which is thus a first light collector 221, on a distal end of the second guide. Similarly, there is shown an exit position, which is thus a second light emitter 223, on distal end of the third guide and an entry position, which is thus a second light collector 225, on a distal end of the fourth guide. The drawing is not to scale. In the following, 'exit position' is used interchangeably with 'light emitter', and 'entry position' is used interchangeably with 'light collector'. The first, second, third and fourth guide are understood to be light guides, such as optical fibres, such as optical waveguides. Furthermore is indicated the first distance d1 between an exit position, corresponding to the first light emitter 219, on the first guide 218 and an entry position, corresponding to the first light collector 221, on the second guide 220. Still further is shown a second distance d2 between an exit position, corresponding to second light emitter 223, on the third guide 222 and an entry position, corresponding to second light collector 225, on the fourth guide 224. Note that the interventional device may be constructed so as to optimise d1 for Reflectance Spectroscopy. In another particular embodiment the interventional device may be constructed so as to optimise d2 for fluorescence spectroscopy. The first distance d1 between the distal ends of the first source and detector light guides is substantially larger than a second distance d2 between the distal ends of the second source and detector light guides.

In a specific embodiment there is provided an optical probe, such as the interventional device 112, is a needle with optical fibers 218, 220, 222, 224 that can be connected to an optical console, such as the optical console 102 depicted in FIG 1. The optical console contains a first light source 104 enabling light to be provided via one of the fibers to the distal end of the optical probe. The scattered light is collected by another fiber and is guided towards the first spectrometer 110 comprising first optical detector 108. The optical console may also contain a second light source 106 being, e.g, a LASER source with a wavelength lower than 450 nm in order to induce autofluorescence in the tissue sample. The obtained data, such as the first and/or second set of measured data are processed by processor 113 using a dedicated algorithm. For instance light is coupled out of the distal tip through at least one fiber, which serves as a source, corresponding to first and/or second light emitter, and the wavelength is swept from e.g. 500-1600 nm or a broadband light source is used. The corresponding wavelength-dependent reflection is measured by at least one other fiber, such as the first and/or second light collector, which may be spatially separated from the source, such as a first distance d1 of at least 0.5, such as at least 1 mm, such as at least 2 mm apart, such as at least 5 mm apart. The amount of reflected light measured at the "detection" fiber, corresponding to a fiber having a distal end being a light collector , is determined by the absorption and scattering properties of the probed structure (i.e., associated tissue sample). From this signal it may be possible to deduce the concentration of chromophores. The autofluorescence is measured through a fiber, corresponding to a fiber having a distal end being a light collector, that is in close vicinity with the excitation fiber, corresponding to a fiber having a distal end being the second light emitter, such as within a second distance d2 being less than 2 mm, such as less than 1 mm, such as less than 0.6 mm, such as less than 0.5 mm, such as less than 0.25 mm from the distal end of the second source light guide, i.e., the light guide having a distal end being the second light emitter. The measured autofluorescence is corrected for scattering and absorption such that the estimated intrinsic fluorescence is obtained.

In a specific embodiment, the apparatus comprises a first light source 104 in the form of a halogen broadband light source with an embedded shutter, an interventional device 112 with four guides and a first spectrometer 110 comprising a first optical detector 108 that can resolve light across a span of wavelengths, such as substantially in the visible and infrared regions of the wavelength spectrum, such as from 400 nm to 1700 nm. The apparatus may furthermore comprise a filter that rejects light for certain wavelengths, such as wavelengths corresponding to a wavelength of light emitted from the LASER source (corresponding to second light source 106), such as a filter blocking light of wavelengths below 405 nm (in case a LASER is used which emits light with a wavelength of 374 nm), which filter may be mounted in front of the first and second spectrometers 110, 111 comprising first and second optical detectors 108, 109. The interventional device 112 has a first guide connected to the light source, the second guide connected to the first optical detector 108. The centre-to-centre distance separation d1 between the distal end of the first light emitter, such as the first source light guide, such as the exit position, corresponding to the first light emitter 219, on the first (emitting) guide 218 and the distal end of the first light collector, such as the first detector light guide, such as the exit position, corresponding to the first light collector 221, on the second (collecting) guide 220 may be in the millimetre range, such as at least 1 mm, such as at least 2 mm, such as 1.8 mm, such as 2.48 mm. All light guides may be low-OH fibres of core diameters in the micron range, such as core diameter of 200 microns. Light fibres containing low-OH, sometimes also called VIS-NIR fibres, are typically suitable for the visible (VIS) and near infrared (NIR) part of the optical spectrum.

In an alternative embodiment a plurality of spectrometers comprising one or more optical detectors are applied, such as two spectrometers with one or more optical detectors that can resolve light in different length regions, such as substantially in the visible and infrared regions of the wavelength spectrum respectively, such as from 400 nm to 1100 nm and from 800 nm to 1700 nm respectively.

Preferably, the optical console allows for the fluorescence excitation wavelength to be changed. This could be accomplished with multiple sources that are switched or multiplexed (e.g. frequency modulated) or with a tunable source. Measuring different fluorescence emission spectra at different excitation wavelengths would provide information that is potentially relevant for differentiating different biomolecular entities, such as for example collagen and elastin (and additionally different types of collagen).

Two-photon fluorescence excitation could also be utilized. This may have the benefits of deeper penetration depth relative to one-photon excitation. The volumes probed with two-photon fluorescence measurements may be more similar to the volumes probed for reflectance measurements in the infrared.

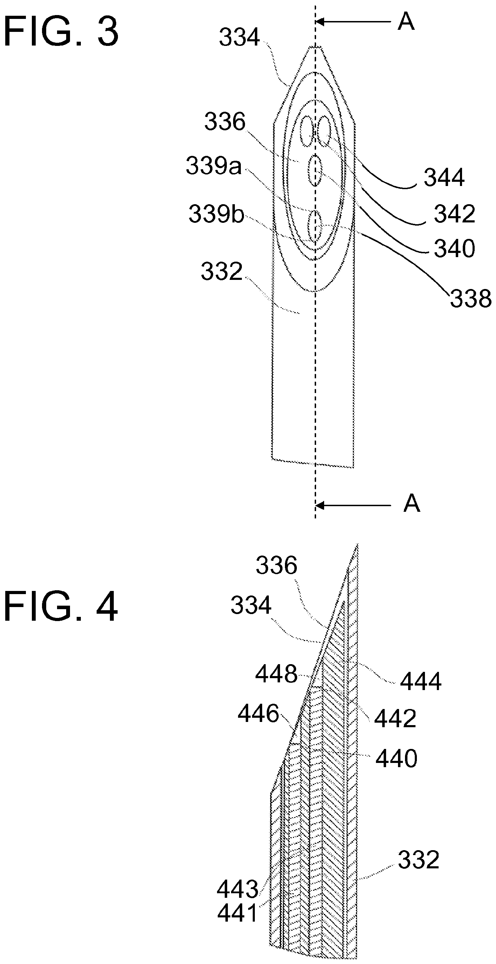

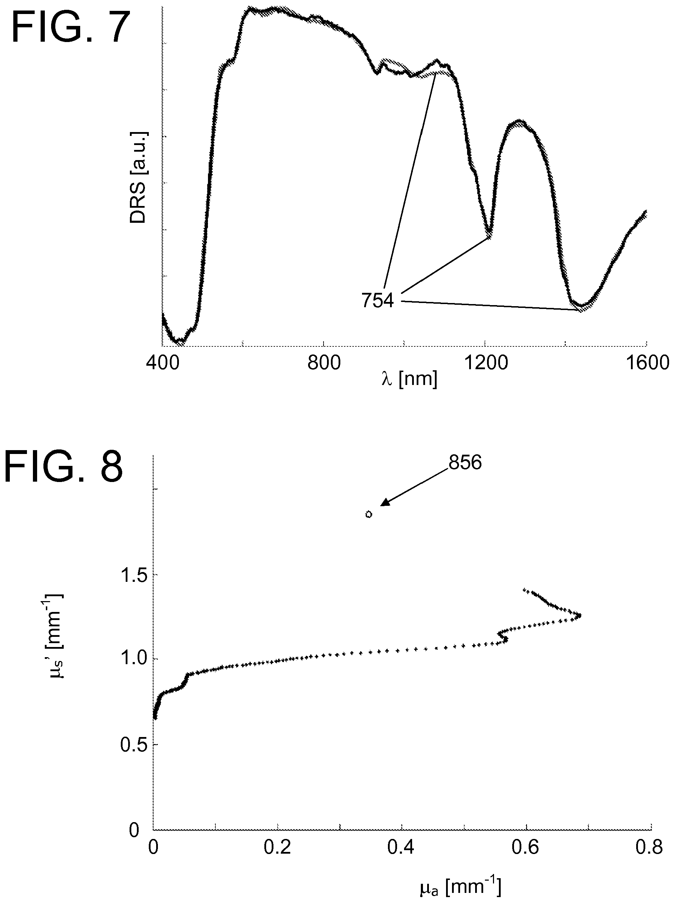

FIGS 3-4 show a schematic depiction of the distal tip of an interventional device according to an embodiment of the invention, where the interventional device is a needle probe used to measure the first and second set of data corresponding, respectively, to a reflectance spectrum and a fluorescence spectrum of the associated tissue sample, such as the spectra shown in FIGS 7, 9-10.

FIG 3 shows a cannula 332 with a cannula bevel 334, a stylet inside the cannula lumen with a stylet endface 336, and holes in the stylet 338, 340, 342, 344 which may each house a light guide. Furthermore is shown top 339a and bottom 339b of the lower hole 338.

FIG 4 shows a sectional view according to the section A indicated in FIG 3, which shows the cannula 332 and the stylet 444, endfaces 440 and 442 of light guides 441 and 443 which are placed in the holes 338 and 340, respectively. An end portion 446, 448 is not filled, due to the oblique cut-off angle of the stylet. The substantially orthogonal cut-off angle of the light-guides may serve to reduce internal reflection.

The top two fibers (placed in holes 342, 344) are each a detector light guide for the fluorescence and DRS measurements (this particular probe uses the same detector light guides for DRS and fluorescence). There are two fibers because different spectrometers comprising detectors are used for the visible and the IR wavelength range. The middle fiber 443 (placed in the hole 340) is the source fiber for the fluorescence excitation light. The bottom fiber 441 (placed in the hole 342) is the source fiber for the DRS light. In this particular embodiment the first distance between the first light emitter and the first light collector is thus given by the distance between the distal end points of the top two fibers (placed in holes 342, 344) and the endface 440 of light guide 441, and the second distance between the second light emitter and the second light collector is given by the distance between the top two fibers (placed in holes 342, 344) and the endface 442 of light guide 443.

Algorithm

In the following an algorithm for extracting information from Reflectance Spectroscopy spectra is described. The inventors of the present application have participated in developing an algorithm that can be used to derive optical tissue properties such as the scattering coefficient and absorption coefficient of different tissue chromophores: e.g. hemoglobin, oxygenated haemoglobin, water, lipid, collagen and elastin from the reflectance spectra. These properties may be different between normal and pathologic tissues.

The algorithm can be described as follows. The spectral fitting will be performed by making use of an analytically derived formula for reflectance spectroscopy which has recently been described in a scientific article featuring the some of the inventors of the present application as authors *"Estimation of lipid and water concentrations in scattering media with diffuse optical spectroscopy from 900 to 1600 nm"*, Nachabé et al., Journal of Biomedical Optics 15(3), 1 (May/June 2010), the article is hereafter referred to as [Nachabé2010a]. Another scientific article by the present authors is given by *"Estimation of biological chromophores using diffuse optical spectroscopy: benefit of extending the UV-VIS wavelength range to include 1000 to 1600 nm"*, R. Nachabé et al., Optics Express 18, 1432-1442 (2010), the article is hereafter referred to as [Nachabé2010b].

The diffuse reflectance model is described in section 2 of [Nachabé2010a], more particularly in section 2.3. The reflectance distribution $R$ is given by

$$R(\rho) = \int_0^\infty R(\rho, z_0)\delta(z_0 - 1/\mu_t')dz_0$$

$$= \frac{d}{4\pi}\left[\frac{1}{\mu_t'}\left(\mu_{eff} + \frac{1}{\tilde{r}_1}\right)\frac{e^{-\mu_{eff}\tilde{r}_1}}{\tilde{r}_1^2} + \left(\frac{1}{\mu_t'} + 2z_b\right)\left(\mu_{eff} + \frac{1}{\tilde{r}_2}\right)\frac{e^{-\mu_{eff}\tilde{r}_2}}{\tilde{r}_2^2}\right]$$

where $\qquad\qquad\qquad\qquad\qquad\qquad\qquad\qquad\qquad\qquad (1)$

$$\tilde{r}_1 = \left[\rho^2 + (1/\mu_t')^2\right]^{1/2}$$

$$\tilde{r}_2 = \left[\rho^2 + ((1/\mu_t') + 2z_b)^2\right]^{1/2}$$

$$\mu_{eff} = \sqrt{3\mu_a[\mu_a + \mu_s(1-g)]}$$

In this formula the three macroscopic parameters describing the probability of interaction with tissue are: the *absorption coefficient* $\mu_a$ and the *scattering coefficient* $\mu_s$ both in cm$^{-1}$ as well as by g which is the mean cosine of the scattering angle. Furthermore, the total reduced attenuation coefficient $\mu_t'$ that gives the total chance for interaction with tissue is given by

$$\mu_t' = \mu_a + \mu_s(1-g). \qquad\qquad\qquad\qquad\qquad (2)$$

The *albedo* a' is the probability of scattering relative to the total probability of interaction we assume a point source at a depth $z_0 = 1/\mu_t'$ and no boundary mismatch hence $z_b = 2/(3\mu_t')$. To simplify some equations we introduce the *reduced scattering coefficient* $\mu_s'$, which is defined as:

$$\mu_s' = \mu_s(1-g). \qquad\qquad\qquad\qquad\qquad\qquad (4)$$

The person skilled in the art will realize that the choice of which scattering coefficient to choose (the reduced scattering coefficient $\mu_s'$ or the traditional scattering coefficient $\mu_s$) is mainly a matter of convenience, since one can easily be transferred into the other (using a reasonable guess or calculation for g). So henceforward it should be understood the any operation involving $\mu_s'$ can also be done with $\mu_s'$ and vice-versa.

Furthermore, we assume that the reduced scattering coefficient can be written as

$$\mu_s{}'(\lambda) = a\lambda^{-b}.$$

(5)

where $\lambda$ is the wavelength and *a* and *b* fixed parameters.

The main absorbing constituents in normal tissue dominating the absorption in the visible and near-infrared range are blood (i.e. hemoglobin), water and lipid.

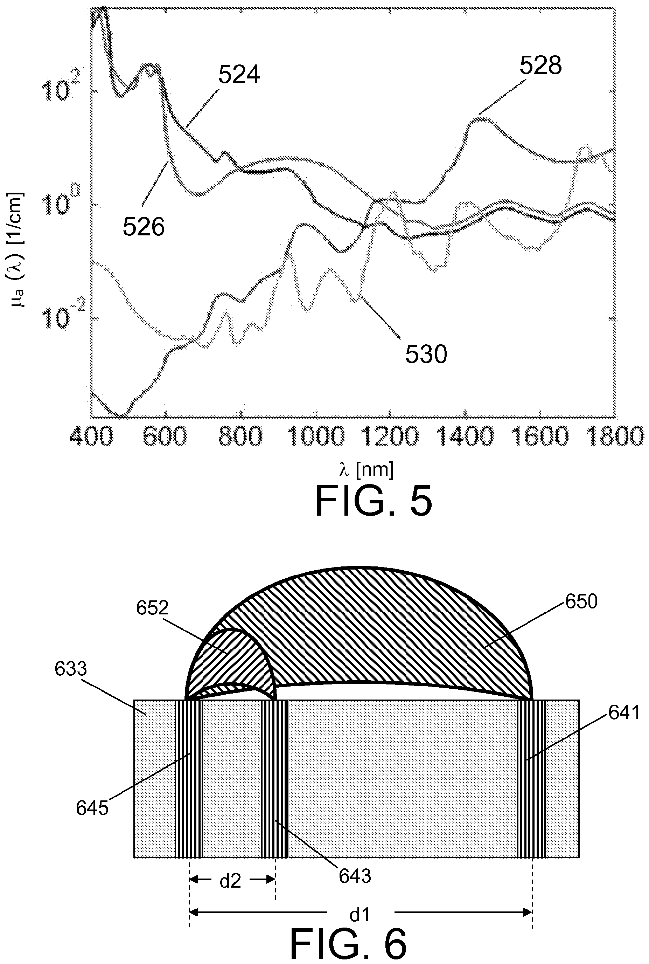

The total absorption coefficient is a linear combination of the absorption coefficients of chromophores in a probed sample, for instance blood, water and lipid as depicted in FIG. 5. Blood dominates the absorption in the visible range, while water and fat dominate in the near infrared range.

For each component the value of that shown in FIG. 5 must be multiplied by its volume fraction. By fitting the above formula while using the power law for scattering it is possible to determine the volume fractions of chromophores present, for example blood, water, lipid, collagen and elastin as well as the scattering coefficient. With this method it is thus possible to translate the measured spectra in physiological parameters that can be used, e.g., to discriminate different tissues.

It is noted that the measurement of the first and second set of data representative of optical spectra of the associated tissue can be carried out in various ways, such as by means of various filter systems in different positions of the optical path, one or more light sources emitting in one or more delimited wavelength bands, or spectrometers (comprising detectors) for different delimited wavelength bands or the detectors being applicable for different delimited wavelength bands. This is understood to be commonly known by the skilled person. It is also possible to modulate the various wavelength bands with different modulation frequencies at the source and demodulate these at the detector, (this technique is described in the published patent application WO2009/153719. Various other modifications can be envisioned without departing from the scope of the invention for instance using more than one spectrometer comprising one or more detectors or using more than one light source with different wavelength band, such as Light Emitting Diodes (LEDs) or LASER sources.

FIG 5 shows absorption spectra of some of the most important chromophores present in the human body namely blood, water and lipid. The graph shows absorption coefficients of deoxygenated haemoglobin (Hb) 524, oxygenated haemoglobin (HbO2) 526, water 528 and lipid 530 as a function of the wavelength. Note that blood dominates the absorption in the visible range, while water and lipids dominate in the near infrared range. The graph has on its first, horizontal axis, the wavelength ($\lambda$, lambda) given in nanometer (nm), and on its second, vertical axis, the absorption coefficient $\mu_a$ (mu_a) given in reciprocal centimetres (1/cm).

According to an embodiment of the invention, the apparatus may comprise a probe, a console and the necessary control and evaluation software.

FIG 6 shows a schematic depiction of the top of the probe 633, which may be an interventional device. This probe contains three optical fibers which are connected to a console, whereby: Fiber A 645 is connected to a spectrometer comprising a detector, Fiber B 643 is connected to a fluorescence excitation light source, e.g., a LASER, and Fiber C 641 is connected to a broadband light source suited for use as a light source for DRS measurements (the person skilled in the art will recognize that it is possible to vary the number of fibers and/or spectrometers comprising detectors e.g. one can use different spectrometers for fluorescence and DRS, or for different spectral ranges). One can use more than one source and/or detector fibers, etc. Light from Fiber C (i.e., from emitted from the distal end of Fiber C corresponding to the first light emitter) which is reflected from the tissue may be collected by Fiber A (i.e., collected at the distal end of Fiber A corresponding to the first light collector) and measured at the spectrometer as a DRS spectrum. Light from Fiber B (ie., emitted from the distal end of Fiber B corresponding to the second light emitter) excites fluorescence in the tissue which is collected by Fiber A (i.e., collected at the distal end of Fiber A which here functions as the second light collector) and measured at the spectrometer as a fluorescence spectrum. In this particular embodiment the same spectrometer turning on either the broadband light or the excitation light source is used. Generalization to more spectrometers, fibers or light sources is straightforward. The source-detector second distance d2 for the fluorescence measurement (i.e., distance between second light emitter and second light collector) is significantly smaller than the corresponding first distance d1 (i.e., distance between first light emitter and first light collector) for the DRS measurement, but the volume 652 probed by fluorescence still significantly overlap with the volume probed by DRS.

FIGS 7-9 show a typical measurement on real tissue. A schematic depiction of the distal end of the probe used is shown in FIGS 3-4. The tip is needle shaped and sharp so that it can be inserted into the tissue. The probe uses the same detector light guides for the DRS and the fluorescence measurements. The visible light and the near infrared light are collected separately and analyzed with different spectrometers (an Andor Technology, DU420A-BRDD for the visible range and an Andor Technology, DU492A-1.7 for the NIR range). For the DRS measurements we used an aventes AvaLight-Hal-S broadband white light source. As fluorescence source a Nichia NDU1113E diode laser with a wavelength of 375 nm was used. To suppress autofluorescence a filter was placed before the visible spectrometer, the filter suppressed all wavelengths below 405 nm. Optical fibers with a diameter of 200 microns and an NA of 0.22 were used. The measurement first distance d1 for the DRS measurement is 1.8 mm, the second distance d2 for the fluorescence measurement 0.32 mm. The measurement was made on excised human breast tissue, directly after the excision procedure.

FIG 7 shows the measured DRS spectrum (black line) of a human breast. The fitted scattering curve is depicted as a grey line 754 in FIG 7. The diffuse reflectance spectra is first used to extract basic tissue properties, namely the reduced scattering and absorption coefficients, $\mu_s$' and $\mu_a$. Since the source-detector distance for the DRS measurement is long enough for diffusion theory to apply, standard theory [Nachabé2010a, Nachabé2010b] can be applied to fit the tissue chromophores and determine the scattering and absorption coefficients, $\mu_s$' and $\mu_a$, at the excitation wavelength $\lambda_x$ and at the emission wavelengths $\lambda_m$ [see FIG 8].

From the DRS fit, for each data point we now have obtained four parameters: $\mu_{sx}$', $\mu_{ax}$, $\mu_{sm}$' and $\mu_{am}$ where the indices $x$ and $m$ stand for the excitation wavelength and the emission wavelength respectively.

The measured fluorescence spectrum $F(\lambda_x, \lambda_m)$ is related to the intrinsic fluorescence $f(\lambda_x, \lambda_m)$ via the equation:

$$f(\lambda_x, \lambda_m) = F(\lambda_x, \lambda_m)/k(\mu_{sx}'(\lambda_x), \mu_{ax}(\lambda_x), \mu_{sm}'(\lambda_m), \mu_{am}(\lambda_m)). \qquad (5)$$

The values of the correction factor $k$ depend strongly on the exact measurement geometries used for the fluorescence measurements. There exists no analytic algorithm to calculate the correction factor $k$ except under very limiting conditions. However we found that it is possible to calculate $k$ using fluorescent Monte Carlo simulations like the ones described in the article "A diffusion theory model of spatially resolved fluorescence from depth-dependent fluorophore concentrations", by D. E. Hyde et al., Phys. Med. Biol. 46, 369-383 (2001) and "Monte-Carlo-based model for the extraction of intrinsic fluorescence from turbid media" by G. M. Palmer et al., J. Biomed. Opt. 13 (2008). The correction factor k is then proportional to the intensity of the emitted light that is collected under the geometry used for the parameter set $\mu_{sx}'(\lambda_x)$, $\mu_{ax}(\lambda_x)$, $\mu_{sm}'(\lambda_m)$ and $\mu_{am}(\lambda_m)$. Since the fitting method described above yields no information on the mean cosine of the scattering angle g, g=0.95 is used for all calculations. This corresponds to a strongly forward directed scattering, which is known to be present in biological tissue.

Since Monte Carlo calculations take a long time these calculations have to be done beforehand and stored in a look-up table. Values of k for values of $\mu_{sx}$', $\mu_{ax}$, $\mu_{sm}$' and $\mu_{am}$ not stored in that look-up table can be interpolated (alternative embodiments may use $\mu_s$' and $\mu_a$ only and set $\mu_{sx}$' and $\mu_{ax}$ to a standard value). This degrades accuracy but will make compiling the look-up table easier, since it limits the number of values significantly. In fact with the simplified look-up table it becomes feasible to determine the look-up table values by measuring well-chosen fluorescent phantoms and interpolating from there. A similar approach for the determination of $\mu_s$' and $\mu_a$ in non-fluorescing samples is described in "Lookup table based inverse model for determining optical properties of turbid media", by N. Rajaram et al., J. Biomed. Opt. 13 (2008). Furthermore, it would be possible to base a look-up table on $\mu_s$ instead of $\mu_s$', i.e. to use the scattering coefficient instead of the reduced scattering coefficient.

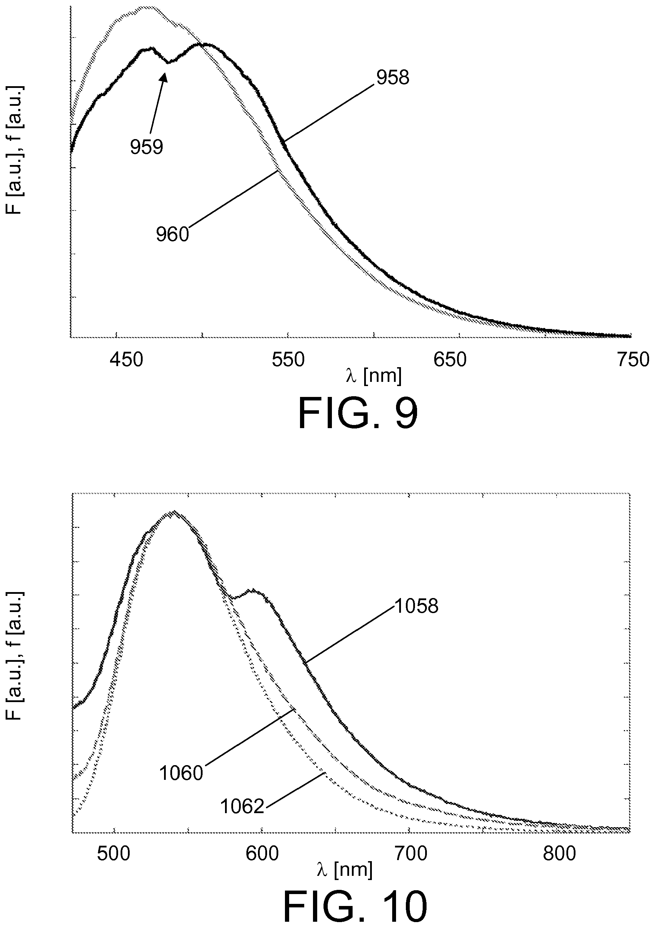

Since the look-up table is pre-calculated, the apparatus only has to apply equation (5) to reconstruct the intrinsic fluorescence spectrum (FIGS 9-10). Therefore only limited calculation/signal processing power is required.

FIG 8 is a graph of the scattering and absorption coefficients, $\mu_s$' and $\mu_a$ at the excitation wavelength (large circle 856) and at the emission wavelengths (the small dots) derived by this method from the fit to the measured DRS spectrum in FIG 7.

FIG 9 shows a measurement of the fluorescence spectrum of the human breast which corresponds to the associated tissue sample relevant for FIG 7. The gray line 960 is the intrinsic fluorescence spectrum determined by the method revealed in this application. Note that the measured fluorescence spectrum (black line 958) shows a prominent dip 959 at a wavelength of $\approx$ 480 nm, which is caused by absorption and which is corrected for in the intrinsic fluorescence spectrum 960.

FIG 10 shows a typical result of a device according to the embodiment described in FIG 6 and the corresponding description. Depicted are the measured fluorescence spectrum 1058 (black, full-drawn line) and the recovered intrinsic fluorescence spectrum 1060 (broken line) in comparison with the 'true' intrinsic fluorescence spectrum 1062 (dotted line) of the pure fluorophore. This measurement is done on a phantom, so the true fluorescence spectrum is well known.

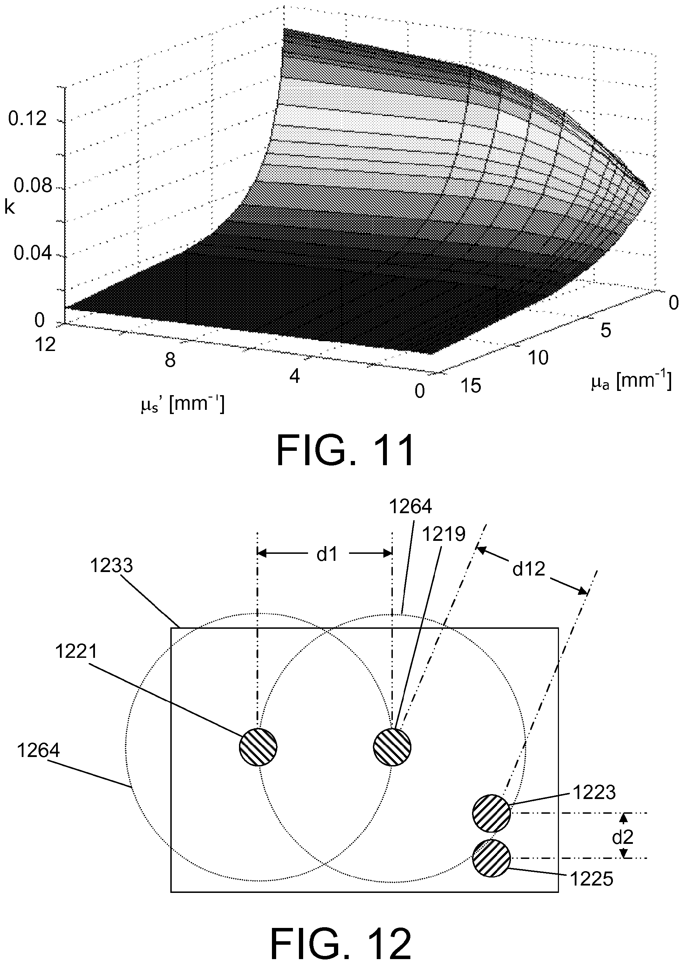

FIG 11 shows an example of a $k(\mu_{sx}'(\lambda_x), \mu_{ax}(\lambda_x), \mu_{sm}'(\lambda_m), \mu_{am}(\lambda_m))$ look-up table. The full look-up table is 4-dimensional of course; the figure shows only a subset for a fixed $\mu_{sx}'(\lambda_x)$-$\mu_{ax}(\lambda_x)$-combination. Calculating the necessary look-up tables takes about 3 weeks on a normal PC.

FIG 12 shows a top of a probe 1233 with various distances illustrated. Furthermore is shown a first distance d1 between a first light emitter 1219 and a first light collector 1221, and a second distance d2 between a second light emitter 1223 and a second light collector 1225. Furthermore, circles 1264 (which are actually spheres, but the depiction is 2-dimensional) encompasses points which are with a radius of d1 from either the first light emitter or the first light collector. In the present example, a smallest distance d12 between the distal ends of the first light emitter and the first light collector, respectively, and the distal ends of the second light emitter and the second light collector is smaller than the first distance d1 between the distal ends of the first light emitter and the first light collector. This can be seen in the figure, since the second light emitter is within one of the circles (spheres) 1264.

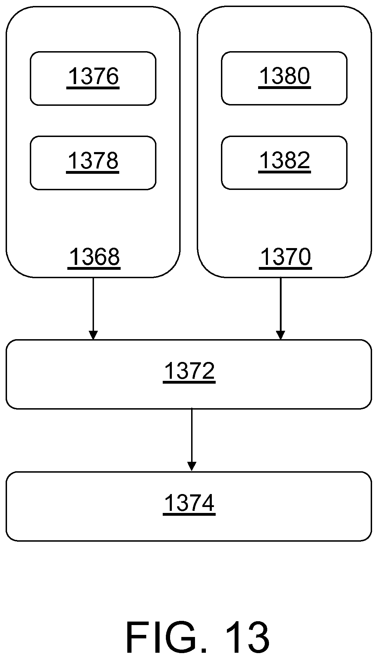

FIG 13 is a flow-chart of a method according to the invention, the method comprising:

- measuring 1368 a first set of data representative of a reflectance spectrum of the associated tissue sample,
- measuring 1370 a second set of data representative of a fluorescence spectrum of the associated tissue sample,
- accessing 1372 a database with a predetermined table of correction factors, and
- determining 1374 a third set of data representative of an intrinsic fluorescence spectrum of the associated tissue sample based on the first and second set of data and the predetermined table of correction factors,

where the measuring 1368 the first set of data comprises

- emitting 1376 photons from a distal end of a first light emitter, and
- collecting 1378 photons at a distal end of first light collector, and

where the measuring 1370 the second set of data comprises

- emitting 1380 photons from a distal end of a second light emitter, and
- collecting 1382 photons at a distal end of second light collector,

wherein a first distance between the distal ends of the first light emitter and the first light collector is substantially larger than a second distance between the distal ends of the second light emitter and the second light collector.

To sum up, in order to improve fluorescence measurements, there is provided an apparatus and, a method and a computer program for optical analysis of an associated tissue sample, the apparatus comprising a spectrometer comprising an optical detector, a light source, a first light emitter arranged for emitting photons into the associated tissue sample, a first light collector arranged for receiving photons from the associated tissue sample, a second light emitter, a second light collector, wherein a reflectance spectrum is obtained via the first light emitter and collector and a fluorescence spectrum is obtained via the second light emitter and collector, and where a first distance d1 between the first light emitter and collector is larger than a second distance d2 between the second light emitter and collector. By combining the data thus obtained, an intrinsic fluorescence spectrum may be obtained.

Although the present invention has been described in connection with the specified embodiments, it should not be construed as being in any way limited to the presented examples. The scope of the present invention is set out by the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

**Claims**

1. An apparatus (100) for optical analysis of an associated tissue sample (116), the apparatus comprising:

    - a spectrometer (110, 111) comprising an optical detector (108, 109),
    - a light source (104, 106),
    - a first light emitter (219) arranged for emitting photons into the associated tissue sample,
    - a first light collector (221) arranged for receiving photons from the associated tissue sample,
    - a second light emitter (223) arranged for emitting photons into the associated tissue sample,
    - a second light collector (225) arranged for receiving photons from the associated tissue sample, and
    where the spectrometer, the light source, the first light emitter (219) and the first light collector (221) are arranged for obtaining a first set of data representative of a spectrum chosen from the group comprising: a reflectance spectrum, a transmission spectrum and an absorption spectrum of the associated tissue sample (116), and
    where the spectrometer, the light source, the second light emitter (223) and the second light collector (225) are arranged for obtaining a second set of data representative of a fluorescence spectrum of the associated tissue sample (116), and the apparatus further comprising
    - a processor (113) arranged for:

- receiving the first set of data, and to determine a wavelength-dependent set of scattering and/or absorption coefficients from the first set of data, and to determine a distortion parameter based on the wavelength-dependent set of scattering and/or absorption coefficients,
- receiving the second set of data, and
- determining a third set of data representative of an intrinsic fluorescence spectrum of the associated tissue sample based on the second set of data and the distortion parameter, wherein a first distance (d1) between the first light emitter (219) and the first light collector (221) is substantially larger than a second distance (d2) between the second light emitter (223) and the second light collector (225), and wherein a first volume (650) of the associated tissue sample which is probed during the measuring of the first set of data substantially overlaps a second volume (652) of the associated tissue sample which is probed during the measuring of the second set of data.

2. An apparatus according to claim 1, the apparatus further comprising:

- a database (114) comprising a predetermined table of correction factors which enables determination of a third set of data being based on the first set of measured data and the second set of measured data,

and the processor (113) further being arranged for:

- accessing the database (114), and

wherein the determining the third set of data representative of an intrinsic fluorescence spectrum of the associated tissue is furthermore based on the predetermined table of correction factors.

3. An apparatus according to claim 1, wherein each one of the first light emitter (219), the first light collector (221), the second light emitter (223) and the second light collector (225) may be a distal end of a light guide.

4. An apparatus according to claim 1, wherein the first light emitter (219), the first light collector (221), the second light emitter (223) and the second light collector (225) are comprised within an interventional device (112).

5. An apparatus according to claim 1, wherein the first distance (d1) between the first light emitter (219) and the first light collector (221) is more than 1 mm.

6. An apparatus according to claim 1, wherein the second distance (d2) between the second light emitter (223) and the second light collector (225) is less than 1 mm.

7. An apparatus according to claim 1, wherein the second light emitter (223) and the second light collector (225) coincide.

8. An apparatus according to claim 1, wherein a smallest distance (d12) between the first light emitter (219) and the first light collector (221), respectively, and the second light emitter (223) and the second light collector (225) is smaller than the first distance (d1) between the first light emitter (219) and the first light collector (221).

9. An apparatus according to claim 1, wherein the first light collector (221) coincides with the second light collector (225).

10. An apparatus according to claim 2, wherein the database (114) further comprises:

- predetermined fourth set of data representative of a predetermined fluorescence spectrum, and wherein the processor is further arranged for
- determining a first parameter being indicative of a concentration of a biomolecule in the associated tissue sample based on the third set of data and the fourth set of data.

11. A method for optical analysis of an associated tissue sample (116), the method comprising:

- measuring (1368) a first set of data representative of a spectrum chosen from the group comprising: a reflectance spectrum, a transmission spectrum and an absorption spectrum of the associated tissue sample,
- determining a wavelength-dependent set of scattering and/or absorption coefficients from the first set of data,
- determining a distortion parameter based on the wavelength-dependent set of scattering and/or absorption coefficients,

- measuring (1370) a second set of data representative of a fluorescence spectrum of the associated tissue sample, and
- determining (1374) a third set of data representative of an intrinsic fluorescence spectrum of the associated tissue sample based on the second set of data and the distortion parameter,

where the measuring (1368) the first set of data comprises emitting (1376) photons from a first light emitter, and collecting (1378) photons at a first light collector, and

where the measuring (1370) the second set of data comprises emitting (1380) photons from a second light emitter, and collecting (1382) photons at a second light collector,

wherein a first distance (d1) between the first light emitter (219) and the first light collector (221) is substantially larger than a second distance (d2) between the second light emitter (223) and the second light collector (225), and wherein a first volume (650) of the associated tissue sample which is probed during the measuring of the first set of data substantially overlaps a second volume (652) of the associated tissue sample which is probed during the measuring of the second set of data.

12. A method according to claim 11 for optical analysis of an associated tissue sample (116), wherein the second volume (652) is substantially a subset of the first volume (650).

**Patentansprüche**

1. Eine Vorrichtung (100) zur optischen Analyse einer zugehörigen Gewebeprobe (116), wobei die Vorrichtung Folgendes umfasst:

- ein Spektrometer (110, 111) mit einem optischen Detektor (108, 109),
- eine Lichtquelle (104, 106),
- einen ersten Lichtemitter (219), der so ausgelegt ist, dass er Photonen in die zugehörige Gewebeprobe emittiert,
- einen ersten Lichtsammler (221), der für den Empfang von Photonen aus der zugehörigen Gewebeprobe ausgelegt ist,
- einen zweiten Lichtemitter (223), der so ausgelegt ist, dass er Photonen in die zugehörige Gewebeprobe emittiert,
- einen zweiten Lichtkollektor (225), der zum Empfang von Photonen aus der zugehörigen Gewebeprobe ausgelegt ist, und

wobei das Spektrometer, die Lichtquelle, der erste Lichtemitter (219) und der erste Lichtkollektor (221) dazu ausgelegt sind, einen ersten Datensatz zu erhalten, der für ein Spektrum repräsentativ ist, das aus der Gruppe ausgewählt ist, die Folgendes umfasst: ein Reflexionsspektrum, ein Transmissionsspektrum und ein Absorptionsspektrum der zugehörigen Gewebeprobe (116), und wobei das Spektrometer, die Lichtquelle, der zweite Lichtemitter (223) und der zweite Lichtkollektor (225) dazu ausgelegt sind, einen zweiten Datensatz zu erhalten, der für ein Fluoreszenzspektrum der zugehörigen Gewebeprobe (116) repräsentativ ist, und wobei die Vorrichtung ferner Folgendes umfasst:

- einen Prozessor (113), der dafür reingerichtet ist:

- den ersten Datensatz zu empfangen, und um einen wellenlängenabhängigen Satz von Streu- und/oder Absorptionskoeffizienten aus dem ersten Datensatz zu bestimmen, und um einen Verzerrungsparameter auf der Grundlage des wellenlängenabhängigen Satzes von Streu- und/oder Absorptionskoeffizienten zu bestimmen,
- den zweiten Datensatzes zu empfangen, und
- einen dritten Datensatzes zu bestimmen, der für ein intrinsisches Fluoreszenzspektrum der zugehörigen Gewebeprobe repräsentativ ist, auf der Grundlage des zweiten Datensatzes und des Verzerrungsparameters, wobei ein erster Abstand (d1) zwischen dem ersten Lichtemitter (219) und dem ersten Lichtkollektor (221) wesentlich größer ist als ein zweiter Abstand (d2) zwischen dem zweiten Lichtemitter (223) und den zweiten Lichtkollektor (225), und wobei ein erstes Volumen (650) der zugehörigen Gewebeprobe, das während der Messung des ersten Datensatzes sondiert wird, im Wesentlichen ein zweites Volumen (652) der zugehörigen Gewebeprobe überlappt , die während der Messung des zweiten Datensatzes sondiert wird.

2. Vorrichtung nach Anspruch 1, wobei die Vorrichtung ferner Folgendes umfasst:

- eine Datenbank (114) mit einer vorbestimmten Tabelle von Korrekturfaktoren, die die Bestimmung eines dritten Datensatzes ermöglicht, der auf dem ersten Satz von Messdaten und dem zweiten Satz von Messdaten basiert,

und der Prozessor (113), der ferner dazu ausgelegt ist:

- auf die Datenbank (114) zuzugreifen , und

wobei die Bestimmung des dritten Datensatzes, der für ein intrinsisches Fluoreszenzspektrum des assoziierten Gewebes repräsentativ ist, ferner auf der vorbestimmten Tabelle von Korrekturfaktoren basiert.

3. Vorrichtung nach Anspruch 1, wobei jeder von dem ersten Lichtemitter (219), dem ersten Lichtkollektor (221), dem zweiten Lichtemitter (223) und dem zweiten Lichtkollektor (225) ein distales Ende eines Lichtleiters sein kann.

4. Vorrichtung nach Anspruch 1, wobei der erste Lichtemitter (219), der erste Lichtkollektor (221), der zweite Lichtemitter (223) und der zweite Lichtkollektor (225) in einer Eingriffsvorrichtung (112) enthalten sind.

5. Vorrichtung nach Anspruch 1, wobei der erste Abstand (d1) zwischen dem ersten Lichtemitter (219) und dem ersten Lichtkollektor (221) mehr als 1 mm beträgt.

6. Vorrichtung nach Anspruch 1, wobei der zweite Abstand (d2) zwischen dem zweiten Lichtemitter (223) und dem zweiten Lichtkollektor (225) weniger als 1 mm beträgt.

7. Vorrichtung nach Anspruch 1, wobei der zweite Lichtemitter (223) und der zweite Lichtkollektor (225) zusammenfallen.

8. Vorrichtung nach Anspruch 1, wobei ein kleinster Abstand (d12) zwischen dem ersten Lichtemitter (219) bzw. dem ersten Lichtkollektor (221) und dem zweiten Lichtemitter (223) und dem zweiten Lichtkollektor (225) kleiner ist als der erste Abstand (d1) zwischen dem ersten Lichtemitter (219) und dem ersten Lichtkollektor (221).

9. Vorrichtung nach Anspruch 1, wobei der erste Lichtkollektor (221) mit dem zweiten Lichtkollektor (225) zusammenfällt.

10. Vorrichtung nach Anspruch 2, wobei die Datenbank (114) ferner Folgendes umfasst:

- vorbestimmter vierter Datensatz, der für ein vorbestimmtes Fluoreszenzspektrum repräsentativ ist, und wobei der Prozessor ferner konfiguriert ist zur
- Bestimmung eines ersten Parameters, der eine Konzentration eines Biomoleküls in der zugehörigen Gewebeprobe anzeigt, auf der Grundlage des dritten und des vierten Datensatzes.

11. Verfahren zur optischen Analyse einer assoziierten Gewebeprobe (116), wobei das Verfahren Folgendes umfasst:

- Messung (1368) eines ersten Datensatzes, der für ein Spektrum repräsentativ ist, das aus der Gruppe ausgewählt wurde, die Folgendes umfasst: ein Reflexionsspektrum, ein Transmissionsspektrum und ein Absorptionsspektrum der zugehörigen Gewebeprobe,
- Bestimmung eines wellenlängenabhängigen Satzes von Streu- und/oder Absorptionskoeffizienten aus dem ersten Datensatz,
- Bestimmung eines Verzerrungsparameters auf der Grundlage des wellenlängenabhängigen Satzes von Streu- und/oder Absorptionskoeffizienten,
- Messung (1370) eines zweiten Datensatzes, der für ein Fluoreszenzspektrum der zugehörigen Gewebeprobe repräsentativ ist, und
- Bestimmung (1374) eines dritten Datensatzes, der repräsentativ für ein intrinsisches Fluoreszenzspektrum der zugehörigen Gewebeprobe ist, basierend auf dem zweiten Datensatz und dem Verzerrungsparameter, wobei die Messung (1368) des ersten Datensatzes das Emittieren (1376) von Photonen von einem ersten Lichtemitter und das Sammeln (1378) von Photonen an einem ersten Lichtkollektor umfasst, und wobei die Messung (1370) des zweiten Datensatzes das Emittieren (1380) von Photonen von einem zweiten Lichtemitter und das Sammeln (1382) von Photonen an einem zweiten Lichtkollektor umfasst, wobei ein erster Abstand (d1) zwischen dem ersten Lichtemitter (219) und dem ersten Lichtkollektor (221) wesentlich größer ist als ein zweiter Abstand (d2) zwischen dem zweiten Lichtemitter

(223) und dem zweiten Lichtkollektor (225), und wobei ein erstes Volumen (650) der zugehörigen Gewebeprobe, die während der Messung des ersten Datensatzes sondiert wird, ein zweites Volumen (652) der zugehörigen Gewebeprobe, die während der Messung des zweiten Datensatzes sondiert wird, im Wesentlichen überlappt.

**12.** Verfahren nach Anspruch 11 zur optischen Analyse einer zugehörigen Gewebeprobe (116), wobei das zweite Volumen (652) im Wesentlichen eine Teilmenge des ersten Volumens (650) ist.

**Revendications**

**1.** Appareil (100) d'analyse optique d'un échantillon tissulaire associé (116), ledit appareil comprenant :

- un spectromètre (110, 111) comprenant un détecteur optique (108, 109),
- une source lumineuse (104, 106),
- un premier émetteur de lumière (219) conçu pour émettre des photons dans l'échantillon tissulaire associé,
- un premier collecteur de lumière (221) conçu pour recevoir des photons de l'échantillon tissulaire associé,
- un second émetteur de lumière (223) conçu pour émettre des photons dans l'échantillon tissulaire associé,
- un second collecteur de lumière (225) conçu pour recevoir des photons de l'échantillon tissulaire associé, et

le spectromètre, la source lumineuse, le premier émetteur de lumière (219) et le premier collecteur de lumière (221) étant conçus pour obtenir un premier ensemble de données représentatives d'un spectre choisi dans le groupe constitué par :
un spectre de réflectance, un spectre de transmission et un spectre d'absorption de l'échantillon tissulaire associé (116), et le spectromètre, la source lumineuse, le second émetteur de lumière (223) et le second collecteur de lumière (225) étant conçus pour obtenir un deuxième ensemble de données représentatives d'un spectre de fluorescence de l'échantillon tissulaire associé (116), et ledit appareil comprenant en outre

- un processeur (113) conçu :

  - pour recevoir le premier ensemble de données, et pour déterminer un ensemble de coefficients de diffusion et/ou d'absorption dépendant de la longueur d'onde à partir du premier ensemble de données, et pour déterminer un paramètre de distorsion en fonction de l'ensemble de coefficients de diffusion et/ou d'absorption dépendant de la longueur d'onde,
  - pour recevoir le deuxième ensemble de données, et
  - pour déterminer un troisième ensemble de données représentatives d'un spectre de fluorescence intrinsèque de l'échantillon tissulaire associé en fonction du deuxième ensemble de données et du paramètre de distorsion,
  dans lequel une première distance (d1), entre le premier émetteur de lumière (219) et le premier collecteur de lumière (221), est sensiblement supérieure à une seconde distance (d2) entre le second émetteur de lumière (223) et le second collecteur de lumière (225), et dans lequel un premier volume (650) de l'échantillon tissulaire associé, lequel est sondé pendant la mesure du premier ensemble de données, chevauche sensiblement un second volume (652) de l'échantillon tissulaire associé, lequel est sondé lors de la mesure du deuxième ensemble de données.

**2.** Appareil selon la revendication 1, ledit appareil comprenant en outre :

- une base de données (114) comprenant une table prédéterminée de facteurs de correction, laquelle permet de déterminer un troisième ensemble de données en fonction du premier ensemble de données mesurées et du deuxième ensemble de données mesurées,

et le processeur (113) étant en outre conçu :

- pour accéder à la base de données (114), et

dans lequel la détermination du troisième ensemble de données représentatives d'un spectre de fluorescence intrinsèque du tissu associé est en outre basée sur la table prédéterminée des facteurs de correction.

**3.** Appareil selon la revendication 1, dans lequel le premier émetteur de lumière (219) et le premier collecteur de lumière (221) et le second émetteur de lumière (223) et le second collecteur de lumière (225) peuvent être une extrémité distale d'un guide de lumière.

4. Appareil selon la revendication 1, dans lequel le premier émetteur de lumière (219), le premier collecteur de lumière (221), le second émetteur de lumière (223) et le second collecteur de lumière (225) sont compris dans un dispositif interventionnel (112).

5. Appareil selon la revendication 1, dans lequel la première distance (d1) entre le premier émetteur de lumière (219) et le premier collecteur de lumière (221) est supérieure à 1 mm.

6. Appareil selon la revendication 1, dans lequel la seconde distance (d2) entre le second émetteur de lumière (223) et le second collecteur de lumière (225) est inférieure à 1 mm.

7. Appareil selon la revendication 1, dans lequel le second émetteur de lumière (223) et le second collecteur de lumière (225) coïncident.

8. Appareil selon la revendication 1, dans lequel la plus petite distance (d12) entre le premier émetteur de lumière (219) et le premier collecteur de lumière (221), respectivement, et le second émetteur de lumière (223) et le second collecteur de lumière (225) est inférieure à la première distance (d1) entre le premier émetteur de lumière (219) et le premier collecteur de lumière (221).

9. Appareil selon la revendication 1, dans lequel le premier collecteur de lumière (221) coïncide avec le second collecteur de lumière (225).

10. Appareil selon la revendication 2, dans lequel la base de données (114) comprend en outre :

   - un quatrième ensemble de données prédéterminées représentatives d'un spectre de fluorescence prédéterminé, et dans lequel le processeur est en outre conçu
   - pour déterminer un premier paramètre indiquant une concentration d'une biomolécule dans l'échantillon tissulaire associé en fonction du troisième ensemble de données et du quatrième ensemble de données.

11. Procédé d'analyse optique d'un échantillon tissulaire associé (116), ledit procédé comprenant :

   - la mesure (1368) d'un premier ensemble de données représentatives d'un spectre choisi dans le groupe constitué par : un spectre de réflectance, un spectre de transmission et un spectre d'absorption de l'échantillon tissulaire associé,
   - la détermination d'un ensemble de coefficients de diffusion et/ou d'absorption dépendant de la longueur d'onde à partir du premier ensemble de données,
   - la détermination d'un paramètre de distorsion en fonction de l'ensemble de coefficients de diffusion et/ou d'absorption dépendant de la longueur d'onde,
   - la mesure (1370) d'un deuxième ensemble de données représentatives d'un spectre de fluorescence de l'échantillon tissulaire associé, et
   - la détermination (1374) d'un troisième ensemble de données représentatives d'un spectre de fluorescence intrinsèque de l'échantillon tissulaire associé en fonction du deuxième ensemble de données et du paramètre de distorsion,
   la mesure (1368) du premier ensemble de données comprenant l'émission (1376) des photons à partir d'un premier émetteur de lumière, et la collecte (1378) des photons au niveau d'un premier collecteur de lumière, et la mesure (1370) du deuxième ensemble de données comprenant l'émission (1380) des photons à partir d'un second émetteur de lumière, et la collecte (1382) des photons au niveau d'un second collecteur de lumière, dans lequel une première distance (d1) entre le premier émetteur de lumière (219) et le premier collecteur de lumière (221) est sensiblement supérieure à une seconde distance (d2) entre le second émetteur de lumière (223) et le second collecteur de lumière (225), et dans lequel un premier volume (650) de l'échantillon tissulaire associé, lequel est sondé lors de la mesure du premier ensemble de données, chevauche sensiblement un second volume (652) de l'échantillon tissulaire associé, lequel est sondé lors de la mesure du deuxième ensemble de données.

12. Procédé selon la revendication 11 destiné à l'analyse optique d'un échantillon tissulaire associé (116), dans lequel le second volume (652) est sensiblement un sous-ensemble du premier volume (650).

FIG. 1

FIG. 2

FIG. 3

334

336

339a

339b

332

A

344

342

340

338

A

FIG. 4

336

334

448

446

443
441

444

442

440

332

FIG. 5

FIG. 6

## FIG. 7

## FIG. 8

FIG. 9

FIG. 10

FIG. 11

FIG. 12

1376

1378

1368

1380

1382

1370

1372

1374

# FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1942335 A
- US 6219566 B1
- US 6571118 B1
- US 2008097174 A1
- WO 2009153719 A

### Non-patent literature cited in the description

- Quantification of in vivo fluorescence decoupled from the effects of tissue optical properties using fiber-optic spectroscopy measurements. *Journal of Biomedical Optics,* 01 January 2010, vol. 15 (6), ISSN 1083.3668, 067006-1, 067006-12
- **NACHABÉ et al.** Estimation of lipid and water concentrations in scattering media with diffuse optical spectroscopy from 900 to 1600 nm. *Journal of Biomedical Optics,* May 2010, vol. 15 (3), 1
- **R. NACHABÉ et al.** Estimation of biological chromophores using diffuse optical spectroscopy: benefit of extending the UV-VIS wavelength range to include 1000 to 1600 nm. *Optics Express,* 2010, vol. 18, 1432-1442
- **D. E. HYDE et al.** A diffusion theory model of spatially resolved fluorescence from depth-dependent fluorophore concentrations. *Phys. Med. Biol.,* 2001, vol. 46, 369-383
- **G. M. PALMER et al.** Monte-Carlo-based model for the extraction of intrinsic fluorescence from turbid media. *J. Biomed. Opt.,* 2008, 13
- **N. RAJARAM et al.** Lookup table based inverse model for determining optical properties of turbid media. *J. Biomed. Opt.,* 2008, 13